# EUROPEAN PATENT APPLICATION

(11) **EP 3 151 007 A1**
(43) Date of publication of application: **05.04.2017**
(21) Application number: 15382477.6
(22) Date of filing: 30.09.2015
(51) Int. Cl.: G01N 33/68, G01N 33/92

(54) **METABOLOMIC SIGNATURE OF DIAGNOSIS AND DISEASE PROGRESSION IN NON-ALCOHOLIC FATTY LIVER DISEASE (NAFLD)**

(71) Applicant: One Way Liver S.L., 48160 Derio-Bizkaia (ES)
(72) Inventor: ALONSO SÁNCHEZ, Cristina, 48160 Derio -Bizkaia (ES); MARTÍNEZ ARRANZ, Ibon, 48160 Derio -Bizkaia (ES); MATO DE LA PAZ, José María, 48160 DERIO- Vizcaya (ES); SANYAL, Arun J., Richmond, VA 23298-0341 (US)
(74) Representative: ABG Patentes, S.L.

(57) **Abstract**

The present invention is directed to a diagnostic method for non-alcoholic fatty liver disease (NAFLD) and to a method for monitoring the progression of the disease based on the determination of the levels of different metabolites. The invention also provides a method for determining the efficacy of an NAFLD therapy and a method for identification of compounds suitable for the treatment of NAFLD.

## Description

### FIELD OF THE INVENTION

The present invention relates to the field of diagnostics and monitorization of disease progression, more in particular the non-invasive diagnosis of non-alcoholic fatty liver disease (NAFLD) and monitorization of progression of the disease, from the early to late fibrosis stage.

### BACKGROUND OF THE INVENTION

Non-alcoholic fatty liver disease (NAFLD) encompasses a wide range of conditions characterized by the build-up of fat in the liver cells in absence of alcohol abuse. At one end of the scale is the relatively harmless simple fatty liver, or steatosis, that does not cause significant liver damage. If left unattended, this condition may progress to more advanced conditions, some of which may be life threatening. Non-alcoholic steatohepatitis (NASH) is a significant development in NAFLD, corresponding to an aggressive condition characterized by swelling and tenderness in the liver. With intense, on-going inflammation a buildup of scar tissue (fibrosis) may form, eventually leading to cirrhosis where irregular bumps, known as nodules, replace the smooth liver tissue and the liver becomes harder. The effect of this, together with continued scarring from fibrosis, means that the liver will run out of healthy cells to support normal functions. This can lead to complete liver failure.

NAFLD is the most common cause of chronic liver disease worldwide. It is considered a direct consequence of the rising global epidemic of obesity and the associated increase in the prevalence of diabetes. Most people with a fatty liver are overweight or obese. As more and more people lead inactive lives and carry extra weight around with them, so the number of cases of NAFLD, in particular NASH is rising.

There is currently no specific laboratory test for NASH, making it extremely difficult to diagnose since it is a silent disease and even people who go on to develop fibrosis and cirrhosis may undergo liver damage for many years before symptoms become apparent.

NAFLD may be suspected in subjects with one or more components of the metabolic syndrome, especially obesity and type 2 diabetes, and elevated serum aminotransferase levels [alanine aminotransferase (ALT) and aspartate aminotransferase (AST)] in the absence of alcohol abuse or other common causes of liver disease. The only widely accepted diagnostic tool for distinguishing NASH from other forms of disease is a liver biopsy. This process involves passing a fine hollow needle through the skin and into the liver, withdrawing a small tissue of sample that is submitted for histological examination. Apart from the obvious discomfort induced by this invasive procedure, assessment is often subjective and prone to sampling error.

Many methods have been described to date in an effort to classify and/or diagnose patients within the main stages of the NAFLD, including steatosis, NASH and cirrhosis.

WO2013113992 and WO2015089102 are describing non-invasive tools using plasma biomarkers for differentiating non-alcoholic steatohepatitis (NASH) from non-alcoholic fatty liver (NAFL), and non-alcoholic fatty liver disease (NAFLD) from normal controls. However, none of these documents refers to the progression of the disease.

Therefore, there is a need for novel methods that to not rely on a liver biopsy for diagnosing and monitoring the progression of NAFLD.

### SUMMARY OF THE INVENTION

The authors of the present invention have identified a series of metabolic markers present in the plasma samples collected at the time of liver biopsy comprising samples from control subjects in the absence of NAFLD, subjects classified with NAFLD with no fibrosis, NAFLD with early stage fibrosis (stage 1-2), and NAFLD with advanced fibrosis (stage 3-4). These metabolic markers can then be used in a rapid non-invasive diagnostic method for diagnosing NAFLD and monitoring its progression, based on the determination of the expression level of each individual metabolite and comparing said level to the level in a reference sample.

Thus, in a first aspect the invention relates to a method for the diagnosis of a non-alcoholic fatty liver disease (NAFLD) in a subject comprising determining in a biological sample of said subject the level(s) of one or more of the metabolic markers as defined in tables 1 and 2, wherein
- increased level(s) of one or more of the metabolic marker(s) defined in table 1 with respect to a reference value and/or
- decreased level(s) of one of more of the metabolic marker(s) defined in table 2 with respect to a reference value
are indicative that the subject suffers from NAFLD.

In a second aspect the invention relates to a method for monitoring the progression of a non-alcoholic fatty liver disease (NAFLD) in a subject comprising determining in a biological sample from said subject the level(s) of one or more of the metabolic markers as defined in tables 1 and 2, wherein
- increased level(s) of one or more of the metabolic marker(s) defined in table 1 with respect to a reference value determined in a sample from the same subject at an earlier time point and/or
- decreased level(s) of one of more of the metabolic marker(s) defined in table 2 with respect to a reference value determined in a sample from the same subject at an earlier time point
are indicative of a progression in the NAFLD.

In a third aspect, the invention relates to a method for determining the efficacy of a therapy for NAFLD comprising determining in a biological sample of a subject suffering from NAFLD and having been treated with said therapy the level(s) of one or more of the metabolic markers as defined in tables 1 and 2, wherein
- decreased level(s) of one or more of the metabolic marker(s) defined in table 1 with respect to a reference value and/or
- increased level(s) of one of more of the metabolic marker(s) defined in table 2 with respect to a reference value
are indicative that the therapy is effective.

In a fourth aspect, the invention relates to a method for the identification of compounds suitable for the treatment of NAFLD comprising determining in a biological sample of a subject suffering from NAFLD and having been treated with a candidate compound the level(s) of one or more of the metabolic markers as defined in table 1 and table 2, wherein
- decreased level(s) of one or more of the metabolic marker(s) defined in table 1 with respect to a reference value and/or
- increased level(s) of one of more of the metabolic marker(s) defined in table 2 with respect to a reference value are indicative that the compound is effective for the treatment of NAFLD.

### DESCRIPTION OF THE DRAWINGS

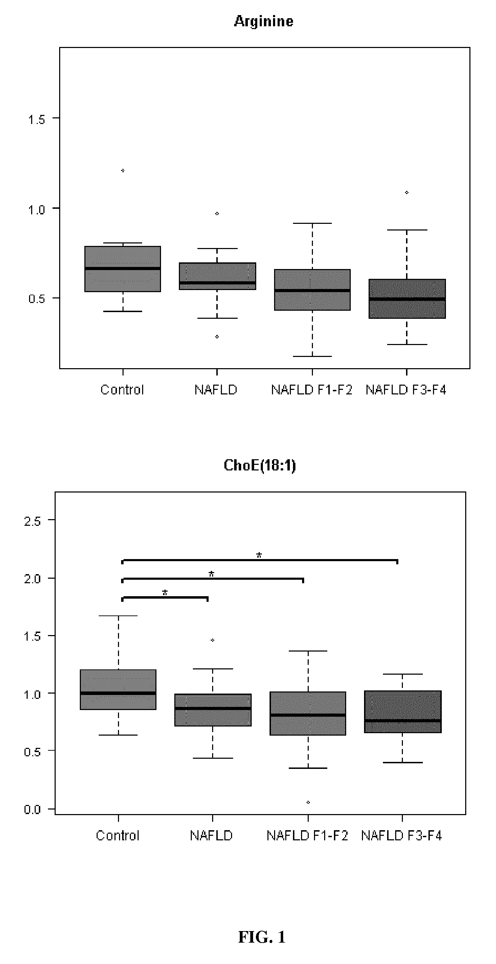

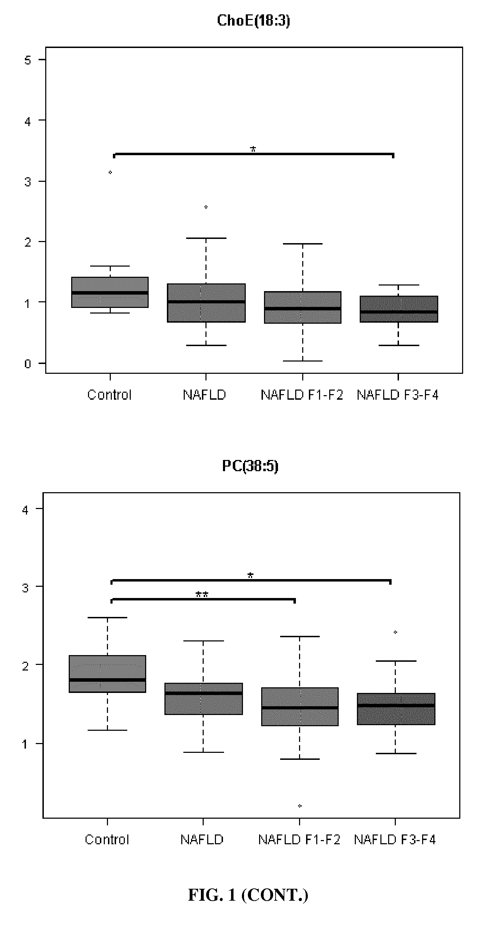

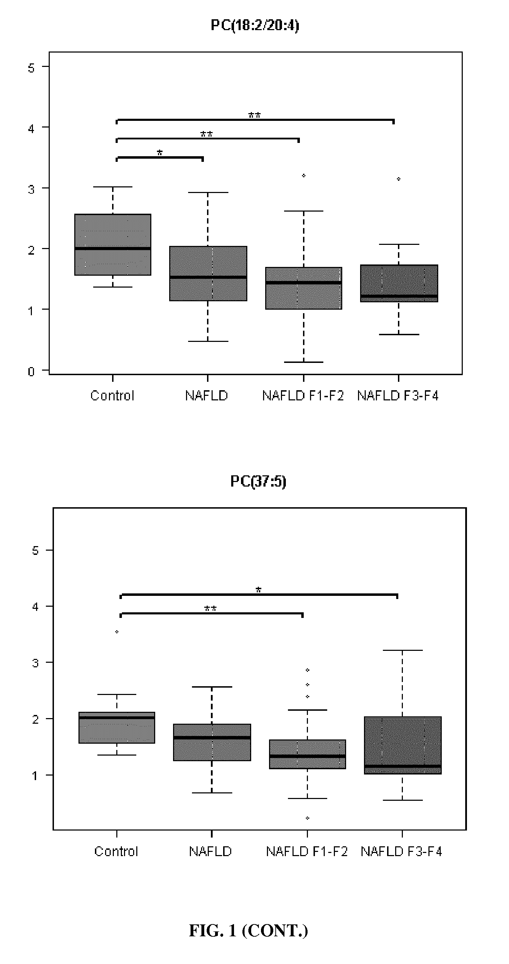

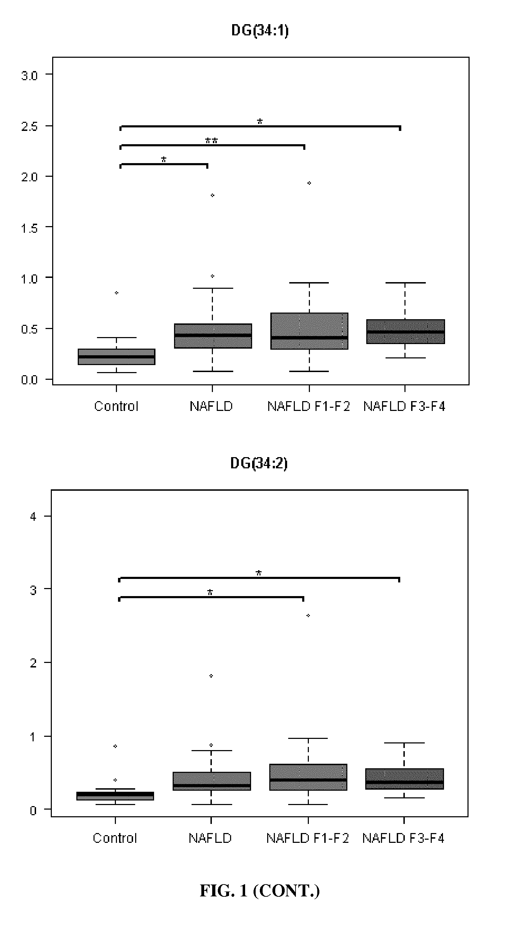

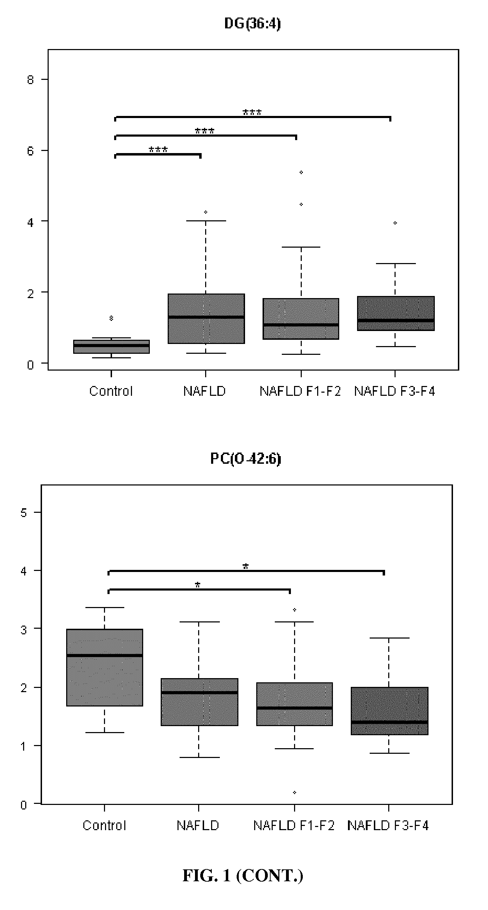

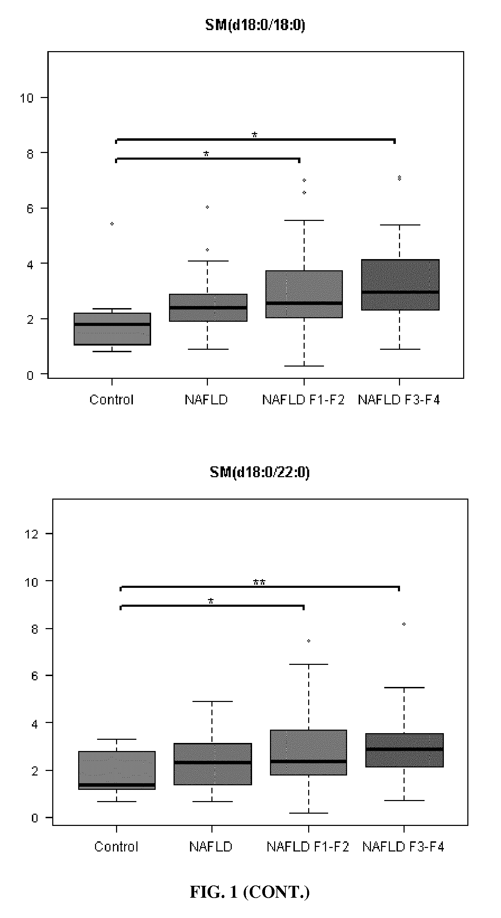

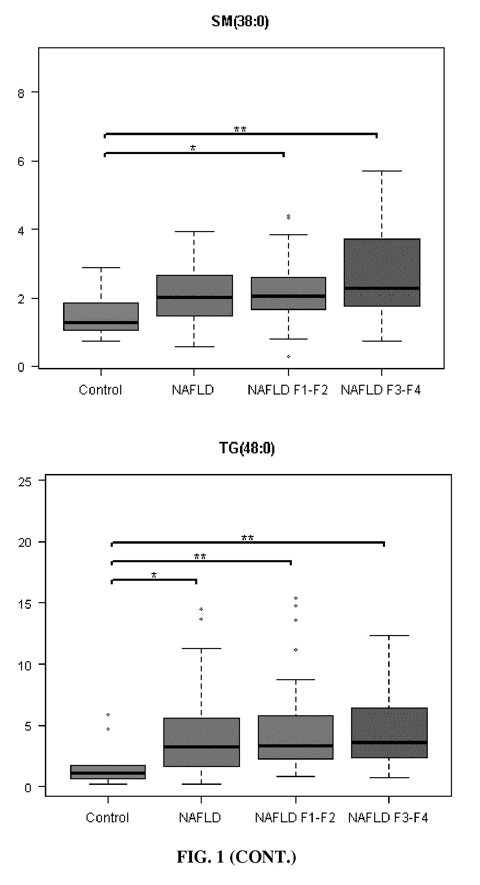

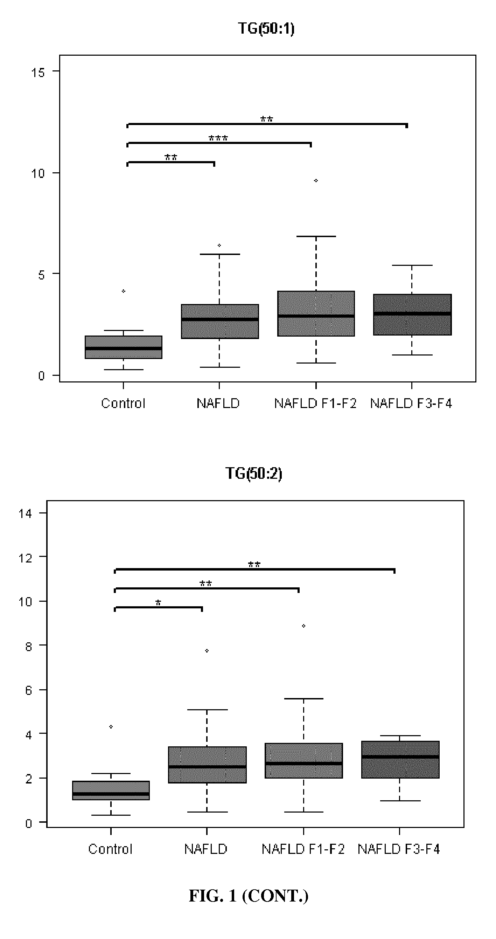

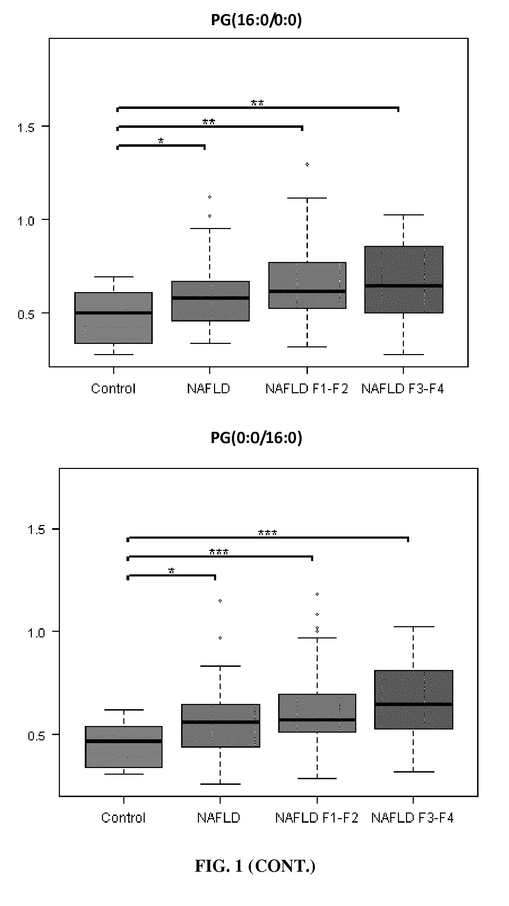

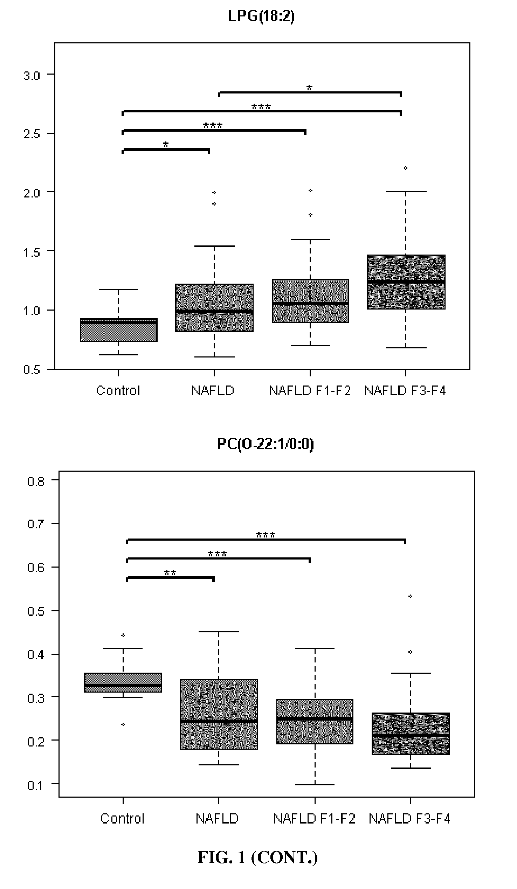

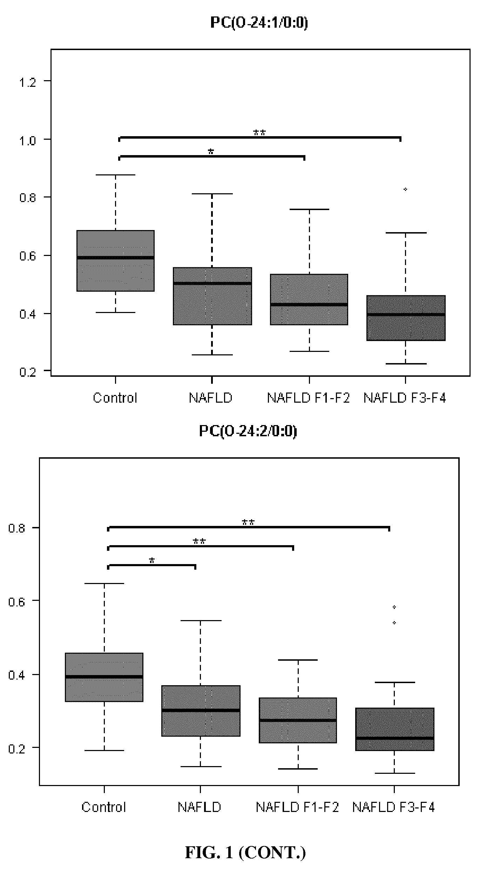

Figure 1. Box-plot graphs of each metabolite in the patient's plasma samples grouped depending on the stage of the disease progression.

### DESCRIPTION OF THE INVENTION

### Diagnostic method of the invention

In a first aspect the invention relates to a method for the diagnosis of a non-alcoholic fatty liver disease (NAFLD) in a subject, hereinafter "diagnostic method of the invention" comprising determining in a biological sample of said subject the level(s) of one or more of the metabolic markers as defined in tables 1 and 2, wherein
- increased level(s) of one or more of the metabolic marker(s) defined in table 1 with respect to a reference value and/or
- decreased level(s) of one of more of the metabolic marker(s) defined in table 2 with respect to a reference value
are indicative that the subject suffers from NAFLD.

The term "diagnosis", as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. It is to be understood that the method, in a preferred embodiment, is a method carried out *in vitro,* i.e. not practiced on the human or animal body. In particular, the term "diagnosis of a non-alcoholic fatty liver disease (NAFLD)" relates to the capacity to identify or detect the presence of NAFLD in a subject. In a particular embodiment, this term also relates to the capacity to identify or detect the presence of a certain degree of NAFLD progression or stage. This diagnosis, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical tools; illustrative, non-limiting examples of said statistical tools include determining confidence intervals, determining the p-value, the Student's t-test or Fisher's discriminant functions, etc. The confidence intervals are preferably at least 90%, at least 95%, at least 97%, at least 98% or at least 99%. The p-value is preferably less than 0.1, less than 0.05, less than 0.01, less than 0.005 or less than 0.0001. The teachings of the present invention preferably allow correctly diagnosing in at least 60%, in at least 70%, in at least 80%, or in at least 90% of the subjects of a determined group or population analyzed.

The term "non-alcoholic fatty liver disease" or "NAFLD", as used herein, refers to a group of conditions having in common the accumulation of fat in the hepatocytes, NAFLD ranges from simple fatty liver (steatosis), to nonalcoholic steatohepatitis (NASH), to cirrhosis (irreversible, advanced scarring of the liver). The term "NAFLD" includes any stage or degree of progression of the disease, including NAFLD without fibrosis, NAFLD with fibrosis stage 1-2 and NAFLD with fibrosis stage 3-4. In a particular embodiment, the NAFLD is selected from the group consisting of NAFLD without fibrosis, NAFLD with fibrosis stage 1-2 and NAFLD with fibrosis stage 3-4.

The terms "NAFLD without fibrosis" or "NAFLD with no fibrosis", as used herein, refer to a NAFLD with fibrosis stage 0.

The terms "NAFLD with fibrosis stage 1-2" or "NAFLD with early stage fibrosis", as used herein, refer to a NAFLD with fibrosis stage 1, 1A, 1B, 1C or 2.

The terms "NAFLD with fibrosis stage 3-4" or "NAFLD with advanced fibrosis", as used herein, refer to a NAFLD with fibrosis stage 3 or 4.

The fibrosis stages are defined by Kleiner et al. (Hepatology 2005, 41(6): 1313-21) and are the following:
- Stage 0: no fibrosis.
- Stage 1: perisinusoidal or periportal fibrosis.
   Score 1A: mild, zone 3, perisinusoidal fibrosis.
   Score 1B: moderate, zone 3, perisinusoidal fibrosis.
   Score 1C: portal/periportal fibrosis.
- Stage 2: perisinusoidal and portal/periportal fibrosis
- Stage 3: bridging fibrosis.
- Stage 4: cirrhosis.

The term "subject" relates to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race. The term "metabolic marker", as used herein, refers to small molecule compounds, such as substrates for enzymes of metabolic pathways, intermediates of such pathways or the products obtained by a metabolic pathway, the occurrence or amount of which is characteristic for a specific situation, for example NAFLD. The metabolic markers useful for the diagnostic method of the invention are those defined in Table 1 and Table 2. The abbreviated names of the lipid metabolites correspond to the lipid family to which it belongs followed by lipid number of the fatty acid side chain. The lipid family is further described by the reference number of said lipid family in the LIPID MAPS structure database (http://www.lipidmaps.org/data/databases.html) using the LIPID MAPS Classification System (Fahy E. et al., Journal of Lipid Research 2009, 50: S9-S14). The lipid number, as the skilled person knows, is a number with the format N:n, where "N" corresponds to the number of carbons in the fatty acid chain and "n" corresponds to the number of double bonds in the fatty acid chain. In the case of glycerophospholipids, since they contain two fatty acids per lipid, when only one number is given it means that both fatty acids are the same. The lipid metabolic markers of Table 1 and Table 2 are intended to refer to any isomer thereof, including structural and geometric isomers. The term "structural isomer", as used herein, refers to any of two or more chemical compounds, having the same molecular formula but different structural formulas. The term "geometric isomer" or "stereoisomer" as used herein refers to two or more compounds which contain the same number and types of atoms, and bonds (i.e., the connectivity between atoms is the same), but which have different spatial arrangements of the atoms, for example cis and trans isomers of a double bond, enantiomers, and diastereomers.

**Table 1. Plasma metabolites up-regulated in NAFLD**

| **Abbreviated name** | **Family name (LIPID MAPS Reference)** |
|---|---|
| DG(34:1) | Diacylglycerols (GL0201) |
| DG(36:4) | Diacylglycerols (GL0201) |
| SM(d18:0/22:0) | Ceramide phosphocholines (sphingomyelins) (SP0301) |
| PG(16:0/0:0) | Monoacylglycerophosphoglycerols (GP0405) |
| TG(48:0) | Triacylglycerols (GL0301) |
| SM(38:0) | Ceramide phosphocholines (sphingomyelins)( SP0301) |
| LPG(18:2) | Monoacylglycerophosphoglycerols (GP0405) |
| DG(34:2) | Diacylglycerols (GL0201) |
| TG(50:1) | Triacylglycerols (GL0301) |
| PG(0:0/16:0) | Monoacylglycerophosphoglycerols (GP0405) |
| TG(50:2) | Triacylglycerols (GL0301) |
| SM(d18:0/18:0) | Ceramide phosphocholines (sphingomyelins)(SP0301) |

**Table 2. Plasma metabolites down-regulated in NAFLD. For arginine, the HMDB (Human Metabolome Database) accession number is provided.**

| **Abbreviated name** | **Family name (LIPID MAPS Reference)/ Amino acid name (HMDB accession number)** |
|---|---|
| PC(37:5) | Diacylglycerophosphocholines (GP0101) |
| ChoE(18:1) | Steryl esters (ST0102) |
| Arginine | 2-Amino-5-guanidinopentanoic acid (HMDB00517) |
| PC(O-22:1/0:0) | Monoalkylglycerophosphocholines (GP0106) |
| PC(38:5) | Diacylglycerophosphocholines (GP0101) |
| PC(O-24:1/0:0) | Monoalkylglycerophosphocholines (GP0106) |
| PC(O-24:2/0:0) | Monoalkylglycerophosphocholines (GP0106) |
| PC(18:2/20:4) | Diacylglycerophosphocholines (GP0101) |
| PC(O-42:6) | 1-alkyl,2-acylglycerophosphocholines (GP0102) |
| ChoE(18:3) | Steryl esters (ST0102) |

In a particular embodiment, the diagnostic method of the invention comprises determining the level of DG(34:1).

In a particular embodiment, the diagnostic method of the invention comprises determining the level of DG(36:4).

In a particular embodiment, the diagnostic method of the invention comprises determining the level of PC(37:5).

In a particular embodiment, the diagnostic method of the invention comprises determining the level of SM(d18:0/22:0).

In a particular embodiment, the diagnostic method of the invention comprises determining the level of ChoE(18:1).

In a particular embodiment, the diagnostic method of the invention comprises determining the level of PG(16:0/0:0).

In a particular embodiment, the diagnostic method of the invention comprises determining the level of TG(48:0).

In a particular embodiment, the diagnostic method of the invention comprises determining the level of arginine.

In a particular embodiment, the diagnostic method of the invention comprises determining the level of PC(O-22:1/0:0).

In a particular embodiment, the diagnostic method of the invention comprises determining the level of SM(38:0).

In a particular embodiment, the diagnostic method of the invention comprises determining the level of LPG(18:2).

In a particular embodiment, the diagnostic method of the invention comprises determining the level of PC(38:5).

In a particular embodiment, the diagnostic method of the invention comprises determining the level of PC(O-24:1/0:0).

In a particular embodiment, the diagnostic method of the invention comprises determining the level of DG(34:2).

In a particular embodiment, the diagnostic method of the invention comprises determining the level of TG(50:1).

In a particular embodiment, the diagnostic method of the invention comprises determining the level of PG(0:0/16:0).

In a particular embodiment, the diagnostic method of the invention comprises determining the level of PC(O-24:2/0:0).

In a particular embodiment, the diagnostic method of the invention comprises determining the level of TG(50:2).

In a particular embodiment, the diagnostic method of the invention comprises determining the level of PC(18:2/20:4).

In a particular embodiment, the diagnostic method of the invention comprises determining the level of SM(d18:0/18:0).

In a particular embodiment, the diagnostic method of the invention comprises determining the level of PC(O-42:6).

In a particular embodiment, the diagnostic method of the invention comprises determining the level of ChoE(18:3).

It will be understood that the method of the invention can be carried out by determining the level of a variable number of the metabolites defined in tables 1 and 2 in the biological sample of the subject under study. For example, the level(s) of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or twenty-two of the markers selected from table 1 and table 2. The determination of levels of combinations of the metabolic markers may allow greater sensitivity and specificity in diagnosing NAFLD.

In a preferred embodiment the metabolic markers of Table 1 and Table 2 correspond to the molecules disclosed in Tables 5 and 6.

The term "sample" or "biological sample", as used herein, refers to biological material isolated from a subject. The biological sample may contain any biological material suitable for detecting the desired biomarker and may comprise cellular and/or non-cellular material from the subject. The sample can be isolated from any suitable biological tissue or fluid such as, for example, liver tissue, blood, blood plasma, serum, urine or cerebral spinal fluid (CSF). Preferably, the samples used for the determination of the level(s) of the metabolic markers are samples which can be obtained using minimally invasive procedures. In a preferred embodiment, the samples are plasma samples. In a more particular embodiment, the samples are EDTA (ethylenediaminetetraacetic acid) plasma samples.

The term "level", as used herein, refers to the quantity of a biomarker detectable in a sample.

It will be understood that the biological sample can be analyzed as such or, alternatively, the metabolites may be first extracted from the sample prior to analysis and then the metabolite extract is then analyzed. If the metabolites are extracted prior to analysis, different extraction methods are available to the skilled person. The selection of one or other extraction method will depend on the class of metabolites/small molecules that are targeted from a particular analysis. Suitable extraction methods include "Extraction of free metabolite pools", "Vapor Phase Extraction", and "Total Metabolite Extraction". The first type of extraction, "Extraction of free metabolite pools", is mainly used in metabolomics research. In this case free intracellular metabolite pools are obtained from a biological sample through methanol- water extraction for polar metabolites, or chloroform, methanol, chloroform/methanol extraction for non-polar metabolites. The second type of extraction, "Vapor Phase Extraction", refers to the extraction of metabolites that are volatile at room temperature. The metabolites are expelled from the biological sample in the vapor phase. These metabolites are either measured directly by connecting the flask or reactor in which the vapors are generated to the analytical instrument or by absorbing first the vapors in charcoal/solvent and then analyzing the acquired solution. The third type of extraction, "Total Metabolite Extraction", refers to the extraction of the free metabolite pools along with the metabolites that have been incorporated in cellular macromolecules, e.g. lipids, proteins etc. The present invention provides extraction of a particular class of metabolites from macromolecules (e.g. amino acids from proteins or sugars from cell wall components). The present invention also provides a combined high-throughput method which extracts all metabolites simultaneously.

Alternatively, the metabolite quantification can be carried out directly in the biological sample. In this case, the sample may be prepared to enhance detectability of the markers. For example, to increase the detectability of markers, a blood serum sample from the subject can be preferably fractionated by, e.g., Cibacron blue agarose chromatography and single stranded DNA affinity chromatography, anion exchange chromatography, affinity chromatography (e.g., with antibodies) and the like. The method of fractionation depends on the type of detection method used. Any method that enriches for the metabolite of interest can be used. Typically, preparation involves fractionation of the sample and collection of fractions determined to contain the biomarkers. Methods of pre- fractionation include, for example, size exclusion chromatography, ion exchange chromatography, heparin chromatography, affinity chromatography, sequential extraction, gel electrophoresis and liquid chromatography. The analytes also may be modified prior to detection. These methods are useful to simplify the sample for further analysis. For example, it can be useful to remove high abundance proteins, such as albumin, from blood before analysis.

In yet another embodiment, a sample can be pre- fractionated by removing proteins that are present in a high quantity or that may interfere with the detection of markers in a sample. Proteins in general may be removed by using conventional techniques such as precipitation using organic solvents such as methanol precipitation, ethanol, acetonitrile, acetone or combinations thereof, in particular, combination of methanol, acetone and acetonitrile, acid precipitation using, for example, trichloroacetic acid or perchloric acid, heat denaturation and any combination of organic solvent, acid and heat precipitation. In the case of a blood, plasma or serum sample, serum albumin or other proteins abundant in serum such as apolipoproteins, glycoproteins, immunoglobulins may obscure the analysis of markers since they are present in a high quantity. Thus, it may be sufficient to remove one or more of the above proteins albumin in order to detect the metabolites or minor proteins. For this purpose, the blood serum or plasma sample can be pre- fractionated by removing serum albumin. Serum albumin can be removed using a substrate that comprises adsorbents that specifically bind serum albumin. For example, a column which comprises, e.g., Cibacron blue agarose (which has a high affinity for serum albumin) or anti-serum albumin antibodies can be used. In yet another embodiment, a sample can be pre-fractionated by isolating proteins that have a specific characteristic, e.g. are glycosylated. For example, a blood serum or plasma sample can be fractionated by passing the sample over a lectin chromatography column (which has a high affinity for sugars). Many types of affinity adsorbents exist which are suitable for pre-fractionating blood serum or plasma samples. An example of one other type of affinity chromatography available to pre-fractionate a sample is a single stranded DNA spin column. These columns bind proteins which are basic or positively charged. Bound proteins are then eluted from the column using eluants containing denaturants or high pH. Thus there are many ways to reduce the complexity of a sample based on the binding properties of the proteins in the sample, or the characteristics of the proteins in the sample.

In yet another embodiment, a sample can be fractionated using a sequential extraction protocol. In sequential extraction, a sample is exposed to a series of adsorbents to extract different types of bio molecules from a sample.

In a particular embodiment, the determination of the level of the one or more metabolic markers is carried out by mass spectrometry. As used herein, "mass spectrometry" (MS analysis) refers to an analytical technique to identify unknown compounds including: (1) ionizing the compounds and potentially fractionating the compounds parent ion formed into daughter ions; and (2) detecting the charged compounds and calculating a mass-to- charge ratio (m/z). The compounds may be ionized and detected by any suitable means. A "mass spectrometer" includes means for ionizing compounds and for detecting charged compounds.

Preferably, mass spectrometry is used in particular gas chromatography coupled to mass spectrometry (GC-MS), liquid chromatography coupled to mass spectrometry (LC-MS), direct infusion mass spectrometry or Fourier transform ion-cyclotrone-resonance mass spectrometry (FT-ICR-MS), capillary electrophoresis coupled to mass spectrometry (CE-MS), high-performance liquid chromatography coupled to mass spectrometry (HPLC-MS), ultra high-performance liquid chromatography coupled to mass spectrometry (UHPLC-MS), supercritical fluid chromatography coupled to mass spectroscopy (SFC-MS), flow injection analysis with mass spectrometry (FIA-MS), including quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time-of-flight mass spectrometry (TOF), of electrospray ionization mass spectrometry (ESI-MS), ESI- MS/MS, ESI- (MS)<n>, matrix-assisted laser desorption ionization time-of-flight mass spectrometry (MALDI-TOF-MS), surface-enhanced laser desorption/ionization time- of-flight mass spectrometry (SELDI-TOFMS), desorption/ionization on silicon (DIOS), secondary ion mass spectrometry (SIMS), quadrupole time-of- flight (Q-TOF), atmospheric pressure chemical ionization mass spectrometry (APCI-MS), APCI- MS/MS, APCI-(MS)<n>, atmospheric pressure photoionization mass spectrometry (APPI- MS), APPI-MS/MS, and APPI-(MS)<n>, quadrupole mass spectrometry, Fourier transform mass spectrometry (FTMS), and ion trap mass spectrometry, where n is an integer greater than zero. Most preferably, LC-MS is used as described in detail below. Said techniques are disclosed in, e.g., Nissen, Journal of Chromatography A, 703, 1995: 37- 57, US 4,540,884 or US 5,397,894.

The above mentioned ionization methods generally produce an ion resulting from the addition of one or more atoms or by cleavage of the molecule. These ions can then be used as surrogate markers for the metabolites used in the method of the invention. The term "surrogate marker" as used herein means a biological or clinical parameter that is measured in place of the biologically definitive or clinically most meaningful parameter.

Typically, the ions result from the addition of a proton or a hydrogen nucleus, [M+H]<+> where M signifies the molecule of interest, and H signifies the hydrogen ion, which is the same as a proton. Some ionization methods will also produce analogous ions. Analogous ions may arise by the addition of an alkaline metal cation, rather than the proton discussed above. A typical species might be [M+Na]<+>, [M+NH4]<+> or [M+K]<+>. The analysis of the ionized molecules is similar irrespective of whether one is concerned with a protonated ion as discussed above or dealing with an added alkaline metal cation. The major difference is that the addition of a proton adds one mass unit (typically called one Dalton), in case of the hydrogen ion (i.e., proton), 23 Daltons in case of sodium18 Daltons in the case of ammonia or 39 Daltons in case of potassium. These additional weights or masses are simply added to the molecular weight of the molecule of interest and the MS peak occurs at the point for the molecular weight of the molecule of interest plus the weight of the ion that has been added. These ionization methods can also produce negative ions. The most common molecular signal is the deprotonated molecule [M-H]<->, in this case the mass is one Dalton lower than the molecular weight of the molecule of interest. In addition, for some compounds it will be produced multiply charged ions. These are of the general identification type of [M+nH]<n+>, where small n identifies the number of additional protons that have been added.

Preferably, the sample (or the eluent when the sample has been fractionated prior to the mass spectrometry) may be introduced into a high resolution mass spectrometer (for example, a LCT Premier™, Waters Corp., Milford, USA) by electrospray ionization, with capillary and cone voltages set in the positive and negative ion modes to 3200 V and 30 V, and 2800 V and 50 V, respectively. An appropriate test mixture of standard compounds may be analyzed before and after the entire set of randomized injection in order to examine the retention time stability, mass accuracy and sensitivity of the system throughout the course of the run.

In another particular embodiment, the biological sample is fractionated by liquid chromatography prior to the determination of the level(s) of the metabolic marker(s). The term "chromatography", as used herein, refers to a method for mixture component separation that relies on differences in the flowing behavior of the various components of a mixture/solution carried by a mobile phase through a support/column coated with a certain stationary phase. Specifically, some components bind strongly to the stationary phase and spend longer time in the support, while other components stay predominantly in the mobile phase and pass faster through the support. The criterion based on which the various compounds are separated through the column is defined by the particular problem being investigated and imposed by the structure, composition and binding capacity of the stationary phase. For example, a stationary phase could be constructed such that the linear and low molecular weight molecules elute faster than the aromatic and high-molecular weight ones. As the components elute from the support, they can be immediately analyzed by a detector or collected for further analysis. A vast number of separation methods, and in particular chromatography methods, are currently available, including Gas Chromatography (GC), Liquid Chromatography (LC), Ion Chromatography (IC), Size-Exclusion Chromatography (SEC), Supercritical-Fluid Chromatography (SFC), Thin-Layer Chromatography (TLC), High Performance Liquid Chromatography (HPLC), Ultra High Performance Liquid Chromatography (UHPLC), and Capillary Electrophoresis (CE). GC can be used to separate volatile compounds or derivatized compounds that, otherwise, are non-volatile compounds. LC is an alternative chromatographic technique useful for separating ions or molecules that are dissolved in a solvent. The principle of GC and LC separation is the same, their main difference lies on the phase in which the separation occurs (gas vs. liquid phase). In addition, GC is used primarily to separate molecules up to 650 atomic units heavy, while, in principle, a LC can separate any molecular weight compounds. Suitable types of liquid chromatography that can be applied in the method of the invention include, without limitation, reverse phase chromatography, normal phase chromatography, affinity chromatography, ion exchange chromatography, hydrophilic interaction liquid chromatography (HILIC), size exclusion chromatography and chiral chromatography. These techniques are well known in the art and can be applied by the person skilled in the art without further ado.

Once the sample has been processed, the first method of the invention involves the determination of the levels of the metabolite in the sample. The expression "determining the levels of a metabolite", as used herein, refers to ascertaining the absolute or relative amount or concentration of the metabolite in the sample. There are many ways to collect quantitative or relational data on metabolites, and the analytical methodology does not affect the utility of metabolite concentrations in predicting phenotype or assessing metabolism. Suitable methods for determining the levels of a given metabolite include, without limitation, refractive index spectroscopy (RI), UltraViolet spectroscopy (UV), fluorescent analysis, radiochemical analysis, Infrared spectroscopy (IR), Nuclear Magnetic Resonance spectroscopy (NMR), Light Scattering analysis (LS), Mass Spectrometry, Pyrolysis Mass Spectrometry, Nephelometry, Dispersive Raman Spectroscopy, gas chromatography combined with mass spectroscopy, liquid chromatography combined with mass spectroscopy, supercritical fluid chromatography combined with mass spectroscopy, MALDI combined with mass spectroscopy, ion spray spectroscopy combined with mass spectroscopy, capillary electrophoresis combined with mass spectrometry, NMR combined with mass spectrometry and IR combined with mass spectrometry.

In a particular embodiment, the levels of the metabolic markers are determined by mass spectrometry. In still more particular embodiment, the biological sample is fractionated by liquid chromatography prior to the determination of the levels of the metabolic markers. In one still more particular embodiment, the liquid chromatography is performed on a C18 column at 40°C. The column may be eluted with a 19 minute gradient using a mobile phase at a flow rate of 140 µL/min initially consisting of 100% solvent A (0.05% formic acid), with a linear increase of solvent B (acetonitrile containing 0.05% formic acid) up to 50% over two minutes, and a linear increase to 100% B over the next 11 min before returning to the initial composition in readiness for the subsequent injection which preceded a 45 s system recycle time. In another still more particular embodiment, the liquid chromatography is performed on a C18 column at 60°C. The column may be eluted with a 17 min linear gradient of solvents A (water, acetonitrile and 10 mM ammonium formate), and B (acetonitrile, isopropanol and 10 mM ammonium formate). The mobile phase, at a flow rate of 400 µL/min, initially consisted of 40% solvent B, increasing up to 100% at 10 minutes. After 5 minutes the mobile phase was reset to the initial composition in readiness for the subsequent injection which preceded a 45 s system recycle time. In another still more particular embodiment, analytes are separated by means of a gradient of solvents A (10 mM ammonium bicarbonate, pH=8.8 adjusted with ammonium hydroxide 28% in water) and B (acetonitrile) with a flow rate of 0.140 µL/min using a gradient starting with 98% of A that decreases linearly to 92% at min 6.5, 80% at min 10, 70% at min 11 and 0% at min 12. After 2 minutes the mobile phase is reset to the initial composition in readiness for the subsequent injection which preceded a 45 s system recycle time.

The diagnostic method of the invention comprises comparing the level(s) of the metabolic marker(s) with a reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value or can be based on a large number of samples, such as from population of subjects of the chronological age matched group, or based on a pool of samples including or excluding the sample to be tested.

The reference value according to the diagnostic method of the invention can be obtained from one or more subjects who do not suffer from NAFLD (i.e., control subjects), of from subjects suffering from NAFLD at any stage, i.e., NAFLD without fibrosis, NAFLD with fibrosis stage 1-2 and NAFLD with fibrosis stage 3-4. Depending on the reference value used the diagnostic method of the invention can allow the classification of a subject into a particular stage of the disease.

Thus, in a particular embodiment, when the reference value is obtained from a subject not suffering from NAFLD, increased level(s) of one or more of the metabolic marker(s) defined in table 1 with respect to said reference value and/or decreased level(s) of one of more of the metabolic marker(s) defined in table 2 with respect to said reference value are indicative that the subject suffers from NAFLD without fibrosis, NAFLD with fibrosis stage 1-2 or NAFLD with fibrosis stage 3-4.

In another particular embodiment, when the reference value is obtained from a subject suffering from NAFLD with no fibrosis, increased level(s) of one or more of the metabolic marker(s) defined in table 1 with respect to said reference value and/or decreased level(s) of one of more of the metabolic marker(s) defined in table 2 with respect to said reference value are indicative that the subject suffers from NAFLD with fibrosis stage 1-2 or NAFLD with fibrosis stage 3-4

In another particular embodiment, when the reference value is obtained from a subject suffering from NAFLD fibrosis stage 1-2, increased level(s) of one or more of the metabolic marker(s) defined in table 1 with respect to said reference value and/or decreased level(s) of one of more of the metabolic marker(s) defined in table 2 with respect to said reference value are indicative that the subject suffers from NAFLD with fibrosis stage 3-4.
- A subject is considered to not suffer from NAFLD if they have not been diagnosed with NAFLD. The diagnosis of NAFLD can be made based on the following criteria: Evidence of steatosis by imaging or histology.
- No secondary cause of fat accumulation in the liver (i.e. alcohol consumption
- No evidence of hepatocellular injury although liver enzyme levels can be elevated in some patients.

A subject is considered to suffer from NAFLD with no fibrosis if they present fibrosis stage 0 as previously defined.

A subject is considered to suffer from NAFLD with fibrosis stage 1-2 if they present fibrosis stage 1, 1A, 1B, 1C or 2 as previously defined.

A subject is considered to suffer from NAFLD with fibrosis stage 3-4 if they present fibrosis stage 3 or 4 as previously defined.

According to the diagnostic method of the invention, the level of a metabolic marker is considered "decreased" when the level of said marker in a sample is lower than its reference value. The level of a marker is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

Likewise, in the context of the diagnostic method of the invention, the level of a marker is considered "increased" when the level of said marker in a sample is higher than its reference value. The level of a marker is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

### Method for monitoring the progression of NAFLD

In a second aspect, the invention relates to a method for monitoring the progression of a non-alcoholic fatty liver disease (NAFLD) in a subject, hereinafter second method of the invention, comprising determining in a biological sample from said subject the level(s) of one or more of the metabolic markers as defined in tables 1 and 2, wherein
increased level(s) of one or more of the metabolic marker(s) defined in table 1 with respect to a reference value determined in a sample from the same subject at an earlier time point and/or
decreased level(s) of one of more of the metabolic marker(s) defined in table 2 with respect to a reference value determined in a sample from the same subject at an earlier time point
are indicative of a progression in the NAFLD.

The terms "NAFLD", "biological sample", "level", "metabolic marker", "increased" and "decreased" have been defined in connection with the diagnostic method of the invention. The metabolic markers defined in Tables 1 and 2, as well as the techniques for determining the level of these markers, have also been defined in connection to the diagnostic method of the invention. The particular and preferred embodiments of the diagnostic method of the invention regarding these terms are also applicable to the second method of the invention.

In a particular embodiment, the NAFLD is NAFLD with no fibrosis, NAFLD with fibrosis stage 1-2 or NAFLD with fibrosis stage 3-4.

The term "subject suffering from NAFLD", as used herein, refers to a subject who has been diagnosed with NAFLD. The diagnosis of NAFLD can be made based on the diagnosis criteria explained in connection with the diagnostic method of the invention.

The term "monitoring the progression", as used herein, refers to the determination of the evolution of the disease in a subject diagnosed with NAFLD, i.e., whether the NAFLD is worsening or whether it is ameliorating.

The term "progression in the NAFLD", as used herein, is understood as a worsening of the disease, i.e., that the disease is progressing to a later stage with respect to the stage at the first time point measured. In a particular embodiment, a progression in the NAFLD means a progression from NAFLD with no fibrosis to NAFLD with fibrosis stage 1-2 or NAFLD with fibrosis stage 3-4. In another particular embodiment, a progression in the NAFLD means a progression from NAFLD with fibrosis stage 1-2 to NAFLD with fibrosis stage 3-4.

In a particular embodiment, the biological sample is plasma.

In a particular embodiment, the second method of the invention comprises determining the level of DG(34:1).

In a particular embodiment, the second method of the invention comprises determining the level of DG(36:4).

In a particular embodiment, the second method of the invention comprises determining the level of PC(37:5).

In a particular embodiment, the second method of the invention comprises determining the level of SM(d18:0/22:0).

In a particular embodiment, the second method of the invention comprises determining the level of ChoE(18:1).

In a particular embodiment, the second method of the invention comprises determining the level of PG(16:0/0:0).

In a particular embodiment, the second method of the invention comprises determining the level of TG(48:0).

In a particular embodiment, the second method of the invention comprises determining the level of arginine.

In a particular embodiment, the second method of the invention comprises determining the level of PC(O-22:1/0:0).

In a particular embodiment, the second method of the invention comprises determining the level of SM(38:0).

In a particular embodiment, the second method of the invention comprises determining the level of LPG(18:2).

In a particular embodiment, the second method of the invention comprises determining the level of PC(38:5).

In a particular embodiment, the second method of the invention comprises determining the level of PC(O-24:1/0:0).

In a particular embodiment, the second method of the invention comprises determining the level of DG(34:2).

In a particular embodiment, the second method of the invention comprises determining the level of TG(50:1).

In a particular embodiment, the second method of the invention comprises determining the level of PG(0:0/16:0).

In a particular embodiment, the second method of the invention comprises determining the level of PC(O-24:2/0:0).

In a particular embodiment, the second method of the invention comprises determining the level of TG(50:2).

In a particular embodiment, the second method of the invention comprises determining the level of PC(18:2/20:4).

In a particular embodiment, the second method of the invention comprises determining the level of SM(d18:0/18:0).

In a particular embodiment, the second method of the invention comprises determining the level of PC(O-42:6).

In a particular embodiment, the second method of the invention comprises determining the level of ChoE(18:3).

In a particular embodiment, the second method of the invention comprises determining the levels of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or twenty-two of the markers selected from table 1 and table 2.

In a preferred embodiment the metabolic markers of Table 1 and Table 2 correspond to the molecules disclosed in Tables 5 and 6.

The second method of the invention involves comparing the levels of one or more metabolic markers in a sample from a subject with a reference value, i.e., the levels of the same marker or markers, determined in a sample from said subject at an earlier time point. In a particular embodiment, the level of the marker in a sample is compared with the level of said marker in the same type of sample.

In a particular embodiment, the determination of the level of the one or more metabolic markers is carried out by mass spectrometry.

In another particular embodiment, the biological sample is fractionated by liquid chromatography prior to the determination of the level(s) of the metabolic marker(s).

### Method for determining the efficacy of a therapy for NAFLD

In a third aspect, the invention relates to a method for determining the efficacy of a therapy for NAFLD, hereinafter third method of the invention, comprising determining in a biological sample of a subject suffering from NAFLD and having been treated with said therapy the level(s) of one or more of the metabolic markers as defined in tables 1 and 2, wherein
- decreased level(s) of one or more of the metabolic marker(s) defined in table 1 with respect to a reference value and/or
- increased level(s) of one of more of the metabolic marker(s) defined in table 2 with respect to a reference value
are indicative that the therapy is effective.

The terms "NAFLD", "biological sample", "level", "metabolic marker", "increased" and "decreased" have been defined in connection with the diagnostic method of the invention. The metabolic markers defined in Tables 1 and 2, as well as the techniques for determining the level of these markers, have also been defined in connection to the diagnostic method of the invention. The particular and preferred embodiments of the diagnostic method of the invention regarding these terms are also applicable to the third method of the invention.

The term "subject suffering from NAFLD" has been defined in connection with the second method of the invention.

In a particular embodiment, the NAFLD is NAFLD with no fibrosis, NAFLD with fibrosis stage 1-2 or NAFLD with fibrosis stage 3-4.

The term "therapy for NAFLD", as used herein, refers to the attempted remediation of a health problem, usually following a diagnosis, or to prevention of the appearance of a health problem. As such, it is not necessarily a cure, i.e. a complete reversion of a disease. Said therapy may or may not be known to have a positive effect on a particular disease. This term includes both therapeutic treatment and prophylactic or preventative measures, in which the object is to prevent or stop (reduce) an undesired physiological change or disorder, such as, NAFLD. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival, disease free survival and symptom free survival, in comparison with the expected survival if treatment is not received. Those subjects needing treatment include those subjects already suffering the condition or disorder, as well as those with the tendency to suffer the condition or disorder or those in which the condition or disorder must be prevented. Illustrative non limitative examples of NAFLD therapies include: administering an agent for treating liver damage, treating associated metabolic conditions such as diabetes and hyperlipidemia, improving insulin resistance, following a balanced and healthy diet, avoiding alcohol, and avoiding unnecessary medications.
In a particular embodiment, the biological sample is plasma.

In a particular embodiment, the third method of the invention comprises determining the level of DG(34:1).

In a particular embodiment, the third method of the invention comprises determining the level of DG(36:4).

In a particular embodiment, the third method of the invention comprises determining the level of PC(37:5).

In a particular embodiment, the third method of the invention comprises determining the level of SM(d18:0/22:0).

In a particular embodiment, the third method of the invention comprises determining the level of ChoE(18:1).

In a particular embodiment, the third method of the invention comprises determining the level of PG(16:0/0:0).

In a particular embodiment, the third method of the invention comprises determining the level of TG(48:0).

In a particular embodiment, the third method of the invention comprises determining the level of arginine.

In a particular embodiment, the third method of the invention comprises determining the level of PC(O-22:1/0:0).

In a particular embodiment, the third method of the invention comprises determining the level of SM(38:0).

In a particular embodiment, the third method of the invention comprises determining the level of LPG(18:2).

In a particular embodiment, the third method of the invention comprises determining the level of PC(38:5).

In a particular embodiment, the third method of the invention comprises determining the level of PC(O-24:1/0:0).

In a particular embodiment, the third method of the invention comprises determining the level of DG(34:2).

In a particular embodiment, the third method of the invention comprises determining the level of TG(50:1).

In a particular embodiment, the third method of the invention comprises determining the level of PG(0:0/16:0).

In a particular embodiment, the third method of the invention comprises determining the level of PC(O-24:2/0:0).

In a particular embodiment, the third method of the invention comprises determining the level of TG(50:2).

In a particular embodiment, the third method of the invention comprises determining the level of PC(18:2/20:4).

In a particular embodiment, the third method of the invention comprises determining the level of SM(d18:0/18:0).

In a particular embodiment, the third method of the invention comprises determining the level of PC(O-42:6).

In a particular embodiment, the third method of the invention comprises determining the level of ChoE(18:3).

In a particular embodiment, the third method of the invention comprises determining the levels of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or twenty-two of the markers selected from table 1 and table 2.

In a preferred embodiment the metabolic markers of Table 1 and Table 2 correspond to the molecules disclosed in Tables 5 and 6.

The third method of the invention involves comparing the levels of one or more metabolic markers in a sample from a subject with a reference value. In a particular embodiment, the reference value is determined in a sample from the same subject before being contacted with the therapy. In another particular embodiment, the reference is determined in a sample from one or more subjects suffering from NAFLD left untreated or having been treated with a control therapy. The term "control therapy", as used herein, refers to a therapy with no effect on NAFLD. Preferably, the control therapy has the same excipient, carrier or vehicle which is used in the therapy whose efficacy for the treatment of NAFLD is to be assessed.

In a particular embodiment, the determination of the level of the one or more metabolic markers is carried out by mass spectrometry.

In another particular embodiment, the biological sample is fractionated by liquid chromatography prior to the determination of the level(s) of the metabolic marker(s).

### Method for the identification of compounds suitable for the treatment of NAFLD

The authors of the present invention have also developed a method for the identification of a compound suitable for the treatment of NAFLD. The identification of a series of metabolites whose levels are increased or decreased with respect to healthy controls allows the screening for compounds in a model of NAFLD which are capable of restoring the levels of the markers to those found in healthy controls.

Thus, in a fourth aspect, the invention relates to a method for the identification of compounds suitable for the treatment of NAFLD, hereinafter fourth method of the invention, comprising determining in a biological sample of a subject suffering from NAFLD and having been treated with a candidate compound the level(s) of one or more of the metabolic markers as defined in table 1 and table 2, wherein
- decreased level(s) of one or more of the metabolic marker(s) defined in table 1 with respect to a reference value and/or
- increased level(s) of one of more of the metabolic marker(s) defined in table 2 with respect to a reference value
are indicative that the compound is effective for the treatment of NAFLD.

The terms "NAFLD", "biological sample", "level", "metabolic marker", "increased" and "decreased" have been defined in connection with the diagnostic method of the invention. The metabolic markers defined in Tables 1 and 2, as well as the techniques for determining the level of these markers, have also been defined in connection to the diagnostic method of the invention. The particular and preferred embodiments of the diagnostic method of the invention regarding these terms are also applicable to the fourth method of the invention.

The term "subject suffering from NAFLD" has been defined in connection with the second method of the invention.

In a particular embodiment, the NAFLD is NAFLD with no fibrosis, NAFLD with fibrosis stage 1-2 or NAFLD with fibrosis stage 3-4.

Examples of suitable animals for use in the screening method of the invention include, but are not limited to, mice, rats, rabbits, monkeys, guinea pigs, dogs and cats. In accordance with this embodiment, the test compound or a control compound is administered (e.g., orally, rectally or parenterally such as intraperitoneally or intravenously) to a suitable animal and the effect on the levels of one or more of the metabolites shown in tables 1 or 2 is determined. Examples of agents include, but are not limited to, nucleic acids (e.g., DNA and RNA), carbohydrates, lipids, proteins, peptides, peptidomimetics, small molecules and other drugs. Agents can be obtained using any of the numerous approaches in combinatorial library methods known in the art. Test compounds further include, for example, antibodies (e.g., polyclonal, monoclonal, humanized, anti-idiotypic, chimeric, and single chain antibodies as well as Fab, F(ab') 2, Fab expression library fragments, and epitope-binding fragments of antibodies). Further, agents or libraries of compounds may be presented, for example, in solution, on beads, chips, bacteria, spores, plasmids or phage.

If the compound is a low-molecular weight compound, then this can be generated by various methods known to the art, preferably synthetically, in particular by combinatorial chemistry, or by biochemical methods, in particular by recombinant expression or purification from biological probes. The compound is of low molecular weight ("small molecules") or the library is composed of molecules with low molecular weight ("small molecule library"). A "small molecule" is defined as a complex collection of compounds, which are produced in a non-biological way, which means which are not produced by recombinant expression, like for instance most protein or peptide libraries. "Small molecules" can be generated by various methods known to the art, but are preferably produced by synthetically, more preferably by combinatory chemistry, to generate a compound library with a maximum chemical diversity within the constraints of predicted attractive drug characteristics. If the compound to be assayed for its suitability for the treatment of NAFLD is a peptide or a peptide library, then these can be generated by various methods known to the art for their use as candidate compounds, but they are preferably produced by biochemical methods, more preferably by recombinant expression in prokaryotic or eukaryotic cells.

The compound to be tested for its suitability for the therapy of NAFLD can be formulated with a pharmaceutically acceptable carrier to produce a pharmaceutical composition, which can be administered to a human or other animal. A pharmaceutically-acceptable carrier can be, for example, water, sodium phosphate buffer, phosphate-buffered saline, normal saline or Ringer's solution or other physiologically-buffered saline, or other solvent or vehicle such as a glycol, glycerol, an oil such as olive oil or an injectable organic ester. A pharmaceutically acceptable carrier can also contain physiologically acceptable compounds that act, for example, to stabilize or increase the absorption of the modulatory compound. One skilled in the art would know that the choice of a pharmaceutically acceptable carrier, including a physiologically acceptable compound, depends, for example, on the route of administration of the composition.

In a particular embodiment, the biological sample is plasma.

In a particular embodiment, the fourth method of the invention comprises determining the level of DG(34:1).

In a particular embodiment, the fourth method of the invention comprises determining the level of DG(36:4).

In a particular embodiment, the fourth method of the invention comprises determining the level of PC(37:5).

In a particular embodiment, the fourth method of the invention comprises determining the level of SM(d18:0/22:0).

In a particular embodiment, the fourth method of the invention comprises determining the level of ChoE(18:1).

In a particular embodiment, the fourth method of the invention comprises determining the level of PG(16:0/0:0).

In a particular embodiment, the fourth method of the invention comprises determining the level of TG(48:0).

In a particular embodiment, the fourth method of the invention comprises determining the level of arginine.

In a particular embodiment, the fourth method of the invention comprises determining the level of PC(O-22:1/0:0).

In a particular embodiment, the fourth method of the invention comprises determining the level of SM(38:0).

In a particular embodiment, the fourth method of the invention comprises determining the level of LPG(18:2).

In a particular embodiment, the fourth method of the invention comprises determining the level of PC(38:5).

In a particular embodiment, the fourth method of the invention comprises determining the level of PC(O-24:1/0:0).

In a particular embodiment, the fourth method of the invention comprises determining the level of DG(34:2).

In a particular embodiment, the fourth method of the invention comprises determining the level of TG(50:1).

In a particular embodiment, the fourth method of the invention comprises determining the level of PG(0:0/16:0).

In a particular embodiment, the fourth method of the invention comprises determining the level of PC(O-24:2/0:0).

In a particular embodiment, the fourth method of the invention comprises determining the level of TG(50:2).

In a particular embodiment, the fourth method of the invention comprises determining the level of PC(18:2/20:4).

In a particular embodiment, the fourth method of the invention comprises determining the level of SM(d18:0/18:0).

In a particular embodiment, the fourth method of the invention comprises determining the level of PC(O-42:6).

In a particular embodiment, the fourth method of the invention comprises determining the level of ChoE(18:3).

In a particular embodiment, the fourth method of the invention comprises determining the levels of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or twenty-two of the markers selected from table 1 and table 2.

In a preferred embodiment the metabolic markers of Table 1 and Table 2 correspond to the molecules disclosed in Tables 5 and 6.

The third method of the invention involves comparing the levels of one or more metabolic markers in a sample from the subject with a reference value. In a particular embodiment, the reference value is determined in a sample from the same subject before being contacted with a candidate compound. In another particular embodiment, the reference is determined in a sample from one or more subjects suffering from NAFLD left untreated or having been treated with a control therapy, preferably the same excipient, carrier or vehicle which is used with the candidate compound.

In a particular embodiment, the determination of the level of the one or more metabolic markers is carried out by mass spectrometry.

In another particular embodiment, the biological sample is fractionated by liquid chromatography prior to the determination of the level(s) of the metabolic marker(s).

### EXAMPLE

### Materials and methods

### Patient's data

| | |
|---|---|
| **Ethnicity** | Non-Hispanic 27/ Hispanic 2/African American 10/Caucasian 64/Asian 1/Unknown 1 |
| **Sex (M/F)** | Female 71/ Male 34 |
| **Age** | 18 - 79 |
| **BMI (kg/m2)** | 21.0 - 53.6 |
| **Diagnosis** | Control 11/NAFLD 29/NAFLD with fibrosis stage (1-2) 43/ NAFLD with fibrosis stage (3-4) 22 |
| **Biopsy (Y/N)** | 105 Y |

### Sample Preparation

Plasma was prepared by incubation patient's venous blood in plasma separator tubes with EDTA for 30 minutes before centrifugation (2500 g, 15 min); supernatants were aliquoted into microtubes and stored at -80°C until metabolomic analysis.

Plasma samples were divided into aliquots and analyzed in three different metabolomic platforms:
Platform 1 - Methanol extract. Proteins were precipitated from the defrosted plasma samples (75 µL) by adding 300 µL of methanol in 1.5 mL microtubes on ice. The extraction solvent was spiked with the following compounds not detected in non-spiked human plasma extracts: NEFA (19:0), tryptophan-d5 (indole-d5), dehydrocholic acid and PC (13:0/0:0). After brief vortex mixing the samples were incubated overnight at -20°C. Supernatants (300 µL) were collected after centrifugation at 16000×g for 15 min and solvent removed. The dried extracts were then reconstituted in 120 µL of methanol, centrifuged (16000×g 5 min), and transferred to vials for UPLC-MS analysis.
Platform 2 - Chloroform/Methanol extract. Proteins were precipitated from the defrosted plasma samples (10 µL) by adding 10 µL of sodium chloride (50 mM) and 110 µL of chloroform/methanol (2:1) in 1.5 mL microtubes on ice. The extraction solvent was spiked with the following compounds not detected in non-spiked human plasma extracts: SM (d18:1/6:0), PE (17:0/17:0), PC (19:0/19:0), TAG (13:0/13:0/13:0), TAG (17:0/17:0/17:0), Cer (d18:1/17:0), ChoE (12:0). After brief vortex mixing the samples were incubated for 1 h at -20°C. After centrifugation at 16000×g for 15 min, 70 µL of the lower organic phase was collected and the solvent removed. The dried extracts were then reconstituted in 100 µL acetronitrile/isopropanol (50:50), centrifuged (16000×g 5 min), and transferred to vials for UPLC-MS analysis.
Platform 3 - Amino acids. 10 µL aliquots of the extracts prepared for platform 1 were transferred to microtubes and derivatized for amino acid analysis using the 6-aminoquinolyl-N-hydroxysuccinimidyl carbamate derivatizing reagent.

### Liquid cromatography - mass spectrometry platforms

A UPLC-single quadrupole-MS amino acid analysis system was combined with two separate UPLC-time-of-flight (TOF)-MS based platforms analyzing methanol and chloroform/methanol plasma extracts. Each platform involves the use of a different UPLC-MS method.
Platform 1: Chromatography was performed on a 1.0 mm i.d. × 100 mm ACQUITY 1.7 µm C18 BEH column (Waters Corp., Milford, MA) using an ACQUITY UPLC system (Waters Corp., Milford, MA). The column was maintained at 40 °C and eluted with a 19 min gradient. The mobile phase, at a flow rate of 140 µL/min, initially consisted of 100% solvent A (0.05% formic acid), with a linear increase of solvent B (acetonitrile containing 0.05% formic acid) up to 50% over two minutes, and a linear increase to 100% B over the next 11 min before returning to the initial composition in readiness for the subsequent injection which preceded a 45 s system recycle time. The volume of sample injected onto the column was 2 µL. The eluent was introduced into the mass spectrometer (LCT-PremierXE, Waters Corp., Milford, MA) by electrospray ionization, with capillary and cone voltages set in the negative ion mode to 2800 and 50 V, respectively. The nebulization gas was set to 600 L/h at a temperature of 350 °C. The cone gas was set to 30 L/h, and the source temperature was set to 120 °C. Centroid data were acquired from m/z 50-1000 using an accumulation time of 0.2 s per spectrum. Platform 2: Chromatography was performed on a 2.1 mm i.d. × 100 mm ACQUITY 1.7 µm C18 BEH column (Waters Corp., Milford, MA) using an ACQUITY UPLC system (Waters Corp., Milford, MA). The column was maintained at 60 °C and eluted with a 17 min linear gradient of solvents A (water, acetonitrile and 10 mM ammonium formate), and B (acetonitrile, isopropanol and 10 mM ammonium formate). The mobile phase, at a flow rate of 400 µL/min, initially consisted of 40% solvent B, increasing up to 100% at 10 minutes. After 5 minutes the mobile phase was reset to the initial composition in readiness for the subsequent injection which preceded a 45 s system recycle time. The volume of sample injected onto the column was 3 µL. The eluent was introduced into the mass spectrometer (Acquity-Xevo G2 QTof, Waters Corp., Milford, MA) by electrospray ionization, with capillary and cone voltages set in the positive ion mode to 3200 and 30 V, respectively. The nebulization gas was set to 1000 L/h at a temperature of 500 °C. The cone gas was set to 30 L/h, and the source temperature was set to 120 °C. Centroid data were acquired from m/z 50-1000 using an accumulation time of 0.2 s per spectrum.
Platform 3: Analytes were separated by means of a gradient of solvents A (10 mM ammonium bicarbonate, pH=8.8 adjusted with ammonium hydroxide 28% in water) and B (acetonitrile). Flow was 0.140 µL/min. Gradient started with 98% of A that decreased linearly to 92% at min 6.5, 80% at min 10, 70% at min 11 and 0% at min 12. After 2 minutes the mobile phase was reset to the initial composition in readiness for the subsequent injection which preceded a 45 s system recycle time. The eluent was introduced into the mass spectrometer (SQD, Waters Corp., Milford, MA) by electrospray ionization, with capillary and cone voltages set in the positive ion mode to 3200 and 30 V, respectively. The nebulization gas was set to 600 L/h at a temperature of 350 °C. The cone gas was set to 10 L/h, and the source temperature was set to 120 °C. Selected ion recording (SIR) was used to analyze desired metabolites. An appropriate test mixture of standard compounds may be analyzed along the entire set of randomized sample injections in order to examine the retention time stability and sensitivity of the system throughout the course of the run which lasted a maximum of 48 h per batch of samples injected.

Online tandem mass spectrometry (MS/MS) experiments for metabolite identification were performed on a Acquity-SYNAPT G2 system and a Xevo G2 QTof system (Waters Corp., Milford, MA) operating in both the positive and negative ion electrospray modes; source parameters were identical to those employed in the profiling experiments, except for the cone voltage which was increased (30-70 V) when pseudo MS/MS/MS data was required. During retention time windows corresponding to the elution of the compounds under investigation, the quadrupole was set to resolve and transmit ions with appropriate mass-to-charge values. The selected ions then traversed an argon-pressurized cell, with a collision energy voltage (typically between 5 and 50 V) applied in accordance with the extent of ion fragmentation required. Subsequent TOF analysis of the fragment ions generated accurate mass generally <3 ppm MS/MS or pseudo MS/MS/MS spectra corrected in real time by reference to leucine enkephalin, infused at 10 µL/min through an independent reference electrospray, sampled every 10 s.

### Data Pre-Processing

All data were pre-processed using the TarkerLynx application manager for MassLynx 4.1 software (Waters Corp., Milford, MA). The complete set of predefined Rt-m/z pairs was fed into the software, which generated associated extracted ion chromatograms (mass tolerance window = 0.05 Da), peak-detected and noise-reduced in both the LC and MS domains. A list of intensities (chromatographic peak areas) was then generated for each sample injection, using the Rt-m/z pairs (retention time tolerance = 6 s) as identifiers. Intra- and interbatch normalization followed the procedure summarized in the paper Martinez-Arranz et al. This process involved (i) internal standard response correction (intrabatch normalization) and (ii) variable specific interbatch single point external calibration using repeat extracts injections of a commercial serum sample (interbatch normalization).

### Data Processing

Univariate statistical analyses were performed calculating group percentage changes and the analysis of variance (ANOVA) (Table 3) and unpaired Student's t-test (or Welch's t test where unequal variances were found) for the comparison among the different groups. The ANOVA provides a statistical test of whether or not the means of several groups are all equal, and therefore generalizes t-test to more than two groups. After selection of significant metabolites in ANOVA, unpaired Student's t-test p-value (or Welch's t test where unequal variances were found) was applied to test for significant differences between specific pairs of means, taken just two at a time for the following comparisons: Control vs. NAFLD; Control vs. NAFLD with stage 1-2 fibrosis; Control vs. NAFLD with stage 3-4 fibrosis; NAFLD vs. NAFLD with stage 1-2 fibrosis; NAFLD vs. NAFLD with stage 3-4 fibrosis; and NAFLD with stage 1-2 fibrosis vs. NAFLD with stage 3-4 fibrosis. Table 4 shows the specific p-value (t-test, or Welch's t test where unequal variances were found) in each comparison between groups: Control vs. NAFLD; Control vs. NAFLD with stage 1-2 fibrosis; Control vs. NAFLD with stage 3-4 fibrosis; NAFLD vs. NAFLD with stage 1-2 fibrosis; NAFLD vs. NAFLD with stage 3-4 fibrosis; and NAFLD with stage 1-2 fibrosis vs. NAFLD with stage 3-4 fibrosis. Tables 5 and 6 show the retention time and mass-to-charge for each metabolite together with the platform used for its identification.

**Table 3. Plasma metabolites detected as relevant for NAFLD diagnosis and monitorization of disease progression.**

| | **Metabolite** | **ANOVA analysis p-value** |
|---|---|---|
| 1 | DG(34:1) | 0.007144227 |
| 2 | DG(36:4) | 0.001341567 |
| 3 | PC(37:5) | 0.009570765 |
| 4 | SM(d18:0/22:0) | 0.005038392 |
| 5 | ChoE(18:1) | 0.007655463 |
| 6 | PG(16:0/0:0) | 0.003774474 |
| 7 | TG(48:0) | 0.004609969 |
| 8 | Arginine | 0.005188243 |
| 9 | PC(O-22:1/0:0) | 0.000784304 |
| 10 | SM(38:0) | 0.00492944 |
| 11 | LPG(18:2) | 0.000118484 |
| 12 | PC(38:5) | 0.004972526 |
| 13 | PC(O-24:1/0:0) | 0.000728181 |
| 14 | DG(34:2) | 0.007068673 |
| 15 | TG(50:1) | 0.00276373 |
| 16 | PG(0:0/16:0) | 0.000520187 |
| 17 | PC(O-24:2/0:0) | 0.000859133 |
| 18 | TG(50:2) | 0.007548278 |
| 19 | PC(18:2/20:4) | 0.001801237 |
| 20 | SM(d18:0/18:0) | 0.001771404 |
| 21 | PC(O-42:6) | 0.002976781 |
| 22 | ChoE(18:3) | 0.005731127 |

**Table 4. p-value (t-test, or Welch's t test where unequal variances were found) in each comparison between groups: Control vs. NAFLD; Control vs. NAFLD with stage 1-2 fibrosis; Control vs. NAFLD with stage 3-4 fibrosis; NAFLD vs. NAFLD with stage 1-2 fibrosis; NAFLD vs. NAFLD with stage 3-4 fibrosis; and NAFLD with stage 1-2 fibrosis vs. NAFLD with stage 3-4 fibrosis.**

| **Metabolite** | **group 1** | **group 2** | **(t-test) p.value** |
|---|---|---|---|
| **Arginine** | Control | NAFLD | 0.2664266 |
| | Control | NAFLD Fibrosis 1-2 | 0.0779268 |
| | Control | NAFLD Fibrosis 3-4 | 0.0580683 |
| | NAFLD | NAFLD Fibrosis 1-2 | 0.1364466 |
| | NAFLD | NAFLD Fibrosis 3-4 | 0.1299630 |
| | NAFLD Fibrosis 1-2 | NAFLD Fibrosis 3-4 | 0.6278385 |
| **ChoE(18:1)** | Control | NAFLD | 0.0480594 |
| | Control | NAFLD Fibrosis 1-2 | 0.0205384 |
| | Control | NAFLD Fibrosis 3-4 | 0.0136775 |
| | NAFLD | NAFLD Fibrosis 1-2 | 0.4862974 |
| | NAFLD | NAFLD Fibrosis 3-4 | 0.3043634 |
| | NAFLD Fibrosis 1-2 | NAFLD Fibrosis 3-4 | 0.6919301 |
| **ChoE(18:3)** | Control | NAFLD | 0.2570753 |
| | Control | NAFLD Fibrosis 1-2 | 0.1004706 |
| | Control | NAFLD Fibrosis 3-4 | 0.0418124 |
| | NAFLD | NAFLD Fibrosis 1-2 | 0.3277719 |
| | NAFLD | NAFLD Fibrosis 3-4 | 0.0614321 |
| | NAFLD Fibrosis 1-2 | NAFLD Fibrosis 3-4 | 0.2313898 |
| **PC(38:5)** | Control | NAFLD | 0.0510805 |
| | Control | NAFLD Fibrosis 1-2 | 0.0070433 |
| | Control | NAFLD Fibrosis 3-4 | 0.0117705 |
| | NAFLD | NAFLD Fibrosis 1-2 | 0.1438645 |
| | NAFLD | NAFLD Fibrosis 3-4 | 0.2696525 |
| | NAFLD Fibrosis 1-2 | NAFLD Fibrosis 3-4 | 0.8296289 |
| **PC(18:2/20:4)** | Control | NAFLD | 0.0416416 |
| | Control | NAFLD Fibrosis 1-2 | 0.0036520 |
| | Control | NAFLD Fibrosis 3-4 | 0.0034028 |
| | NAFLD | NAFLD Fibrosis 1-2 | 0.1576812 |
| | NAFLD | NAFLD Fibrosis 3-4 | 0.1471988 |
| | NAFLD Fibrosis 1-2 | NAFLD Fibrosis 3-4 | 0.8079783 |
| **PC(37:5)** | Control | NAFLD | 0.0629892 |
| | Control | NAFLD Fibrosis 1-2 | 0.0070954 |
| | Control | NAFLD Fibrosis 3-4 | 0.0398956 |
| | NAFLD | NAFLD Fibrosis 1-2 | 0.0732507 |
| | NAFLD | NAFLD Fibrosis 3-4 | 0.5370764 |
| | NAFLD Fibrosis 1-2 | NAFLD Fibrosis 3-4 | 0.5315405 |
| **DG(34:1)** | Control | NAFLD | 0.0238206 |
| | Control | NAFLD Fibrosis 1-2 | 0.0093175 |
| | Control | NAFLD Fibrosis 3-4 | 0.0108387 |
| | NAFLD | NAFLD Fibrosis 1-2 | 0.9025759 |
| | NAFLD | NAFLD Fibrosis 3-4 | 0.9555379 |
| | NAFLD Fibrosis 1-2 | NAFLD Fibrosis 3-4 | 0.8171147 |
| **DG(34:2)** | Control | NAFLD | 0.0619881 |
| | Control | NAFLD Fibrosis 1-2 | 0.0133990 |
| | Control | NAFLD Fibrosis 3-4 | 0.0271194 |
| | NAFLD | NAFLD Fibrosis 1-2 | 0.5113229 |
| | NAFLD | NAFLD Fibrosis 3-4 | 0.8528101 |
| | NAFLD Fibrosis 1-2 | NAFLD Fibrosis 3-4 | 0.5738282 |
| **DG(36:4)** | Control | NAFLD | 0.0006519 |
| | Control | NAFLD Fibrosis 1-2 | 0.0000638 |
| | Control | NAFLD Fibrosis 3-4 | 0.0001342 |
| | NAFLD | NAFLD Fibrosis 1-2 | 0.9318555 |
| | NAFLD | NAFLD Fibrosis 3-4 | 0.8236123 |
| | NAFLD Fibrosis 1-2 | NAFLD Fibrosis 3-4 | 0.8765000 |
| **PC(O-42:6)** | Control | NAFLD | 0.0600212 |
| | Control | NAFLD Fibrosis 1-2 | 0.0239876 |
| | Control | NAFLD Fibrosis 3-4 | 0.0120041 |
| | NAFLD | NAFLD Fibrosis 1-2 | 0.4248579 |
| | NAFLD | NAFLD Fibrosis 3-4 | 0.1722584 |
| | NAFLD Fibrosis 1-2 | NAFLD Fibrosis 3-4 | 0.4672081 |
| **SM(d18:0/18:0)** | Control | NAFLD | 0.2251601 |
| | Control | NAFLD Fibrosis 1-2 | 0.0290411 |
| | Control | NAFLD Fibrosis 3-4 | 0.0148057 |
| | NAFLD | NAFLD Fibrosis 1-2 | 0.1130112 |
| | NAFLD | NAFLD Fibrosis 3-4 | 0.0593624 |
| | NAFLD Fibrosis 1-2 | NAFLD Fibrosis 3-4 | 0.4654921 |
| **SM(d18:0/22:0)** | Control | NAFLD | 0.1500244 |
| | Control | NAFLD Fibrosis 1-2 | 0.0116676 |
| | Control | NAFLD Fibrosis 3-4 | 0.0067634 |
| | NAFLD | NAFLD Fibrosis 1-2 | 0.1435830 |
| | NAFLD | NAFLD Fibrosis 3-4 | 0.0688026 |
| | NAFLD Fibrosis 1-2 | NAFLD Fibrosis 3-4 | 0.5254976 |
| **SM(38:0)** | Control | NAFLD | 0.0583798 |
| | Control | NAFLD Fibrosis 1-2 | 0.0155154 |
| | Control | NAFLD Fibrosis 3-4 | 0.0029800 |
| | NAFLD | NAFLD Fibrosis 1-2 | 0.5632071 |
| | NAFLD | NAFLD Fibrosis 3-4 | 0.0885623 |
| | NAFLD Fibrosis 1-2 | NAFLD Fibrosis 3-4 | 0.1708204 |
| **TG(48:0)** | Control | NAFLD | 0.0119398 |
| | Control | NAFLD Fibrosis 1-2 | 0.0011582 |
| | Control | NAFLD Fibrosis 3-4 | 0.0045356 |
| | NAFLD | NAFLD Fibrosis 1-2 | 0.6352944 |
| | NAFLD | NAFLD Fibrosis 3-4 | 0.7539985 |
| | NAFLD Fibrosis 1-2 | NAFLD Fibrosis 3-4 | 0.8912461 |
| **TG(50:1)** | Control | NAFLD | 0.0061042 |
| | Control | NAFLD Fibrosis 1-2 | 0.0005644 |
| | Control | NAFLD Fibrosis 3-4 | 0.0012295 |
| | NAFLD | NAFLD Fibrosis 1-2 | 0.3748761 |
| | NAFLD | NAFLD Fibrosis 3-4 | 0.5642821 |
| | NAFLD Fibrosis 1-2 | NAFLD Fibrosis 3-4 | 0.7279453 |
| **TG(50:2)** | Control | NAFLD | 0.0160682 |
| | Control | NAFLD Fibrosis 1-2 | 0.0032279 |
| | Control | NAFLD Fibrosis 3-4 | 0.0049545 |
| | NAFLD | NAFLD Fibrosis 1-2 | 0.6109896 |
| | NAFLD | NAFLD Fibrosis 3-4 | 0.7911912 |
| | NAFLD Fibrosis 1-2 | NAFLD Fibrosis 3-4 | 0.7516152 |
| **PG (16:0/0:0)** | Control | NAFLD | 0.0473555 |
| | Control | NAFLD Fibrosis 1-2 | 0.0017523 |
| | Control | NAFLD Fibrosis 3-4 | 0.0062301 |
| | NAFLD | NAFLD Fibrosis 1-2 | 0.1172868 |
| | NAFLD | NAFLD Fibrosis 3-4 | 0.2576226 |
| | NAFLD Fibrosis 1-2 | NAFLD Fibrosis 3-4 | 0.8284562 |
| **PG(16:0)** | Control | NAFLD | 0.0149914 |
| | Control | NAFLD Fibrosis 1-2 | 0.0006427 |
| | Control | NAFLD Fibrosis 3-4 | 0.0001630 |
| | NAFLD | NAFLD Fibrosis 1-2 | 0.3006560 |
| | NAFLD | NAFLD Fibrosis 3-4 | 0.0858548 |
| | NAFLD Fibrosis 1-2 | NAFLD Fibrosis 3-4 | 0.3946067 |
| **LPG(18:2)** | Control | NAFLD | 0.0125673 |
| | Control | NAFLD Fibrosis 1-2 | 0.0003678 |
| | Control | NAFLD Fibrosis 3-4 | 0.0000550 |
| | NAFLD | NAFLD Fibrosis 1-2 | 0.5517349 |
| | NAFLD | NAFLD Fibrosis 3-4 | 0.0392727 |
| | NAFLD Fibrosis 1-2 | NAFLD Fibrosis 3-4 | 0.0695723 |
| **PC(O-22:1/0:0)** | Control | NAFLD | 0.0067443 |
| | Control | NAFLD Fibrosis 1-2 | 0.0004281 |
| | Control | NAFLD Fibrosis 3-4 | 0.0004776 |
| | NAFLD | NAFLD Fibrosis 1-2 | 0.3811318 |
| | NAFLD | NAFLD Fibrosis 3-4 | 0.1731200 |
| | NAFLD Fibrosis 1-2 | NAFLD Fibrosis 3-4 | 0.4308419 |
| **PC(O-24:1/0:0)** | Control | NAFLD | 0.0539377 |
| | Control | NAFLD Fibrosis 1-2 | 0.0142990 |
| | Control | NAFLD Fibrosis 3-4 | 0.0043092 |
| | NAFLD | NAFLD Fibrosis 1-2 | 0.3923780 |
| | NAFLD | NAFLD Fibrosis 3-4 | 0.1060591 |
| | NAFLD Fibrosis 1-2 | NAFLD Fibrosis 3-4 | 0.2822438 |
| **PC(O-24:2/0:0)** | Control | NAFLD | 0.0496541 |
| | Control | NAFLD Fibrosis 1-2 | 0.0086326 |
| | Control | NAFLD Fibrosis 3-4 | 0.0081645 |
| | NAFLD | NAFLD Fibrosis 1-2 | 0.1506693 |
| | NAFLD | NAFLD Fibrosis 3-4 | 0.1769431 |
| | NAFLD Fibrosis 1-2 | NAFLD Fibrosis 3-4 | 0.7474286 |

**Table 5. Plasma metabolites up-regulated in NAFLD. Platform used for identification, retention time, mass-to-charge, adduct and molecular formula is shown.**

| **bbreviated name** | **Family name (LIPID MAPS Reference)** | **Platform** | **Retention time (min)** | **Mass-to-charge (Da)** | **Adduct** | **Molecular Formula** |
|---|---|---|---|---|---|---|
| DG(34:1) | Diacylglycerols (GL0201) | 2 | 6.07 | 617.5121 | [M+Na]+ | C₃₇H₇₀O₅ |
| DG(36:4) | Diacylglycerols (GL0201) | 2 | 5.54 | 639.4964 | [M+Na]+ | C₃₉H₆₈O₅ |
| SM(d18:0/22:0) | Ceramide phosphocholines (sphingomyelins) (SP0301) | 2 | 6.37 | 789.6850 | [M+H]+ | C₄₅H₉₃N₂O₆P |
| PG(16:0/0:0) | Monoacylglycerophosphoglycerols (GP0405) | 1 | 6.51 | 483.2723 | [M-H]- | C₂₂H₄₅O₉P |
| TG(48:0) | Triacylglycerols (GL0301) | 2 | 8.47 | 824.7707 | [M+NH4]+ | C₅₁H₉8O₆ |
| SM(38:0) | Ceramide phosphocholines (sphingomyelins)( SP0301) | 2 | 5.91 | 761.6537 | [M+H]+ | C₄₃H₈₉N₂O₆P |
| LPG(18:2) | Monoacylglycerophosphoglycerols (GP0405) | 1 | 5.77 | 507.2723 | [M-H]- | C₂₄H₄₅O₉P |
| DG(34:2) | Diacylglycerols (GL0201) | 2 | 5.65 | 615.4964 | [M+Na]+ | C₃₇H₆₈O₅ |
| TG(50:1) | Triacylglycerols (GL0301) | 2 | 8.46 | 850.7864 | [M+NH4]+ | C₅₃H₁₀₀O₆ |
| PG(0:0/16:0) | Monoacylglycerophosplioglycerols (GP0405) | 1 | 6.90 | 483.2723 | [M-H]- | C₂₂H₄₅O₉P |
| TG(50:2) | Triacylglycerols (GL0301) | 2 | 8.20 | 848.7707 | [M+NH4]+ | C₅₃H₉₈O₆ |
| SM(d18:0/18:0) | Ceramide phosphocholines (sphingomyelins)( SP0301) | 2 | 5.39 | 733.6224 | [M+H]+ | C₄₁H₈₅N₂O₆P |

**Table 6. Plasma metabolites down-regulated in NAFLD. Platform used for identification, retention time, mass-to-charge, adduct and molecular formula is shown. For arginine, the HMDB (Human Metabolome Database) accession number is provided.**

| **Abbreviated name** | **Family name (LIPID MAPS Reference)/ Amino acid name (HMDB accession number)** | **Platform** | **Retention time (min)** | **Mass-to- charge (Da)** | **Adduct** | **Molecular Formula** |
|---|---|---|---|---|---|---|
| PC(37:5) | Diacylglycerophosphocholines (GP0101) | 2 | 4.35 | 794.5700 | [M+H]+ | C₄₅H₈₀NO₈P |
| ChoE(18:1) | Steryl esters (ST0102) | 2 | 8.50 | 668.6346 | [M+NH4]+ | C₄₅H₇₈O₂ |
| Arginine | 2-Amino-5-guanidinopentanoic acid (HMDB00517) | 3 | 6.04 | 174.1117 | [M_{derivatized}+H]⁺ | C₆H₁₄N₄O₂ |
| PC(O-22:1/0:0) | Monoalkylglycerophosphocholines (GP0106) | 1 | 9.67 | 608.4292 | [M+CO2H]- | C₃₀H₆₂NO₆P |
| PC(38:5) | Diacylglycerophosphocholines (GP0101) | 2 | 4.65 | 808.5856 | [M+H]+ | C₄₆H₈₂NO₈P |
| PC(O-24:1/0:0) | Monoalkylglycerophosphocholines (GP0106) | 1 | 11.37 | 636.4605 | [M+ CO2H]- | C₃₂H₆₆NO₆P |
| PC(O-24:2/0:0) | Monoalkylglycerophosphocholines (GP0106) | 1 | 10.04 | 634.4449 | [M+ CO2H]- | C₃₂H₆₄NO₆P |
| PC(18:2/20:4) | Diacylglycerophosphocholines (GP0101) | 2 | 4.18 | 806.5700 | [M+H]+ | C₄₆H₈₀NO₇P |
| PC(O-42:6) | 1-alkyl,2-acylglycerophosphocholines (GP0102) | 2 | 5.53 | 848.6533 | [M+H]+ | C₅₀H₉₀NO₇P |
| ChoE(18:3) | Steryl esters (ST0102) | 2 | 7.97 | 664.6033 | [M+NH4]+ | C₄₅H₇₄O₂ |

## Claims

1. A method for the diagnosis of a non-alcoholic fatty liver disease (NAFLD) in a subject comprising determining in a biological sample of said subject the level(s) of one or more of the metabolic markers as defined in tables 1 and 2, wherein
increased level(s) of one or more of the metabolic marker(s) defined in table 1 with respect to a reference value and/or
decreased level(s) of one of more of the metabolic marker(s) defined in table 2 with respect to a reference value
are indicative that the subject suffers from NAFLD.

2. A method for monitoring the progression of a non-alcoholic fatty liver disease (NAFLD) in a subject comprising determining in a biological sample from said subject the level(s) of one or more of the metabolic markers as defined in tables 1 and 2, wherein
increased level(s) of one or more of the metabolic marker(s) defined in table 1 with respect to a reference value determined in a sample from the same subject at an earlier time point and/or
decreased level(s) of one of more of the metabolic marker(s) defined in table 2 with respect to a reference value determined in a sample from the same subject at an earlier time point
are indicative of a progression in the NAFLD.

3. A method for determining the efficacy of a therapy for NAFLD comprising determining in a biological sample of a subject suffering from NAFLD and having been treated with said therapy the level(s) of one or more of the metabolic markers as defined in tables 1 and 2, wherein
decreased level(s) of one or more of the metabolic marker(s) defined in table 1 with respect to a reference value and/or
increased level(s) of one of more of the metabolic marker(s) defined in table 2 with respect to a reference value
are indicative that the therapy is effective.

4. A method for the identification of compounds suitable for the treatment of NAFLD comprising determining in a biological sample of a subject suffering from NAFLD and having been treated with a candidate compound the level(s) of one or more of the metabolic markers as defined in table 1 and table 2, wherein
decreased level(s) of one or more of the metabolic marker(s) defined in table 1 with respect to a reference value and/or
increased level(s) of one of more of the metabolic marker(s) defined in table 2 with respect to a reference value are indicative that the compound is effective for the treatment of NAFLD.

5. The method according to claims 3 or 4 wherein the reference value is determined
a) in a sample from the same subject before being contacted with the therapy or with the candidate compound or
b) in a sample from one or more subjects suffering from NAFLD left untreated or having been treated with a control therapy.

6. The method according to any of claims 1 to 5, wherein the NAFLD is NAFLD with no fibrosis, NAFLD with early stage fibrosis (stage 1-2) or NAFLD with advanced fibrosis (stage 3-4).

7. The method according to any of claims 1 to 6 wherein the biological sample is plasma.

8. The method according to any of claims 1 to 7 comprising analyzing the levels of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or twenty-two of the markers selected from table 1 and table 2.

9. The method according to any of claims 1 to 8 wherein the determination of the level of the one or more metabolic markers is carried out by mass spectrometry.

10. The method according to any of claims 1 to 9 wherein the biological sample is fractionated by liquid chromatography prior to the determination of the level(s) of the metabolic marker(s).
